# EUROPEAN PATENT SPECIFICATION

(11) **EP 4 429 629 B1**
(45) Date of publication and mention of the grant of the patent: **06.05.2026**
(21) Application number: 22817094.0
(22) Date of filing: 25.10.2022
(51) Int. Cl.: A61K 8/46, A61Q 5/02, A61Q 5/12, A61K 8/891, A61K 8/892, A61K 8/898, A61K 8/73, A61Q 5/00

(54) **HAIR COMPOSITION COMPRISING A CATIONIC CELLULOSE AND A SILICONE COMPOUND**
HAARZUSAMMENSETZUNG ENTHALTEND KATIONISCHE ZELLULOSE UND EINE SILIKONVERBINDUNG
COMPOSITION CAPILLAIRE COMPRENANT UNE CELLULOSE CATIONIQUE ET UNE SILICONE

(30) Priority: 09.11.2021 WO PCT/CN2021/129512; 15.12.2021 EP 21214577
(43) Date of publication of application: 18.09.2024
(73) Proprietor: Unilever IP Holdings B.V., 6708 WH Wageningen (NL); Unilever Global IP Limited, Wirral, Merseyside CH62 4ZD (GB)
(72) Inventor: AO, Mingqi, 6708 WH Wageningen (NL); LE, Yingyi, 6708 WH Wageningen (NL); PI, Yingying, 6708 WH Wageningen (NL)
(74) Representative: Unilever Patent Group
(86) International application number: PCT/EP2022/079820
(87) International publication number: WO 2023/083609

(56) References cited:
- EP-A2- 0 674 898
- US-B1- 6 706 258

## Description

### Field of the Invention

This invention relates to hair care compositions, more particularly to hair care compositions comprising a cationic cellulose and a small sized silicone compound. The present invention also relates to a method of delivering oil control sensory during topical application to the hair or scalp.

### Background of the Invention

Hair care compositions are formulated for specific purposes, such as cleansing or conditioning benefits or a combination of the two. Preferred hair care composition will provide more benefits than simple cleansing and conditioning. Such compositions typically comprise one or more cleansing surfactants which generally aid in cleaning the hair and the scalp free of undesirable soil, particles and fatty matter.

Scalp hair becomes soiled due to its contact with the surrounding environment and from sebum secreted from the hair follicles. The build- up of sebum and environmental soiling can cause the hair to have a dirty or greasy feel, and an unattractive appearance. In order to ameliorate these effects, it is necessary to shampoo the hair with regularity. Sebum is naturally present on scalp but is often seen as a negative indicator of beauty and health through association with greasy hair or even a suggested association with more serious conditions such as seborrheic dermatitis.

Dandruff control is an important aspect of hair cleansing and care. Anti-dandruff benefit has been provided through hair care compositions including shampoos and hair conditioners. Dandruff is an issue that affects many people globally. The condition is manifested by the shedding of clumps of dead skin cells from the scalp. These are white in colour and provide an aesthetically displeasing appearance. A factor that contributes to dandruff is certain members of the Malassezia yeasts. To combat these, anti-dandruff products have included certain anti-dandruff agents which have anti-fungal activity. These anti-dandruff agents remove (or at least reduce the level of) the Malassezia from the scalp and provide moderately effective treatment of the dandruff condition. Such a product performs as a hair cleansing shampoo or as a hair conditioner while mitigating the causes of dandruff.

US6706258 B1 (Unilever) discloses a shampoo comprising sodium lauryl ether sulphate 2EO (8.0%), Jaguar C13S (0.3%) and silicone emulsion (2.5%) and silicone microemulsion (6%). However, the silicone emulsion is introduced as BY22-048 from Toray Silicone Co, emulsion (60% a.i.) of dimethicone (silicone viscosity 1 million cst, average silicone particle size 0.5 micron=500nm), and the silicone microemulsion is introduced as DC2-1870 from Dow Coming Ltd., a microemulsion (25% a.i.) of dimethiconol (silicone viscosity 60000 million cst, average silicone particle size 0.04 micron=40nm) in anionic/nonionic surfactant. It does not disclose the silicone compound having mean particle size from 150 to 450nm.

EP0674898 A2 (Unilever) discloses a shampoo comprising from 2 to 35% surfactant, from 0.01 to 10% of a microemulsion of particles of a high viscosity silicone having a particle size of <0.15microns and 0.01 to 10% of a cationic deposition polymer.

The present inventors focused their attention on the above-mentioned problems. There remains a need to improve the oil control sensory, especially for the sensory experience of using antidandruff products.

### Summary of the Invention

The inventors of the present invention surprisingly found that inclusion of a combination of a cationic cellulose and a small sized silicone with specific weight ratio in a hair care composition, preferably an anti-dandruff shampoo composition, can deliver desirable sensory which is oil control on hair or scalp, preferably reducing greasiness.

In a first aspect, the present invention is directed to a hair care composition comprising:
i) a cleansing surfactant comprising an anionic surfactant;
ii) a cationic cellulose; and
iii) a silicone having mean particle size from 150 to 450nm;
wherein the weight ratio of the amount of said silicone compound to the amount of said cationic cellulose is from 4.3:1 to 10:1; wherein the mean particle size of the silicone compound is measured by means of a laser light scattering technique.

In a second aspect, present invention is directed to a non-therapeutic method of treating the hair or scalp comprising the step of applying a composition of the first aspect onto the scalp or hair.

In a third aspect, the present invention is directed to a non-therapeutic method of delivering oil control sensory comprising the step of applying the hair care composition of the first aspect onto hair or scalp surfaces of an individual followed by rinsing the surfaces with water.

In a fourth aspect, the present invention is directed to non-therapeutic use of a composition of the first aspect for delivering oil control sensory on hair or scalp.

All other aspects of the present invention will more readily become apparent upon considering the detailed description and examples which follow.

### Detailed Description of the Invention

### Hair Care Composition

"Hair care composition", as used herein, is meant to include a composition for topical application to hair and/or scalp of mammals, especially humans. Such a composition may be generally classified as leave-on or rinse off, and includes any product applied to a human body for also improving appearance, cleansing, odor control or general aesthetics. The composition of the present invention can be in the form of a liquid, lotion, cream, foam, scrub, gel, or bar. Non-limiting examples of such compositions include leave-on hair lotions, creams, and rinse-off shampoos, conditioners, shower gels, or toilet bar. The composition of the present invention is preferably a rinse-off composition, especially preferred being a shampoo or a conditioner and most preferably a shampoo.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final hair care composition, unless otherwise specified.

It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### Cationic cellulose

The hair care composition of the present invention comprises a cationic cellulose.

Cationic cellulose is available, for example, from the Dow Chemical Company in their UCARE JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Dow Chemical Company under the tradename Polymer LM-200. Most preferred is Polyquaternium 10, especially Polyquaternium 10 with a molecular weight (weight average by GPC) in the range 0.5 to 3 million Daltons and charge density in the range 0.5 to 3 meq/g. The preferred Polyquaternium 10 is available, for example, as UCARE^{™} JR30M from Dow Chemical.

Preferably the composition comprises cationic cellulose in an amount of from 0.2 to 1%, more preferably 0.3 to 0.8%, furthermore preferably from 0.3 to 0.6% by weight of the composition.

### Silicone compound

The hair care composition of the present invention comprises a silicone compound having mean particle size from 150 to 450nm.

The silicone compound of the present invention is emulsified droplets of silicone oil, which has a mean droplet diameter (D50) of from 150 to 450nm,
preferably from 150 to 300nm, most preferably from 150 to 250nm.

The mean droplet diameter of a silicone oil may be measured by means of a laser light scattering technique, for example using a 2600D Particle Sizer from Malvern Instruments.

Suitable silicone compounds include polysiloxanes, in particular polydimethylsiloxane which have the CTFA designation dimethicone. Also suitable for use in compositions of 20 this invention (particularly shampoos and conditioners) are polydimethylsiloxanes having hydroxyl end groups, which have the CTFA designation dimethiconol. Another class of silicones which may be used are functionalized silicones such as amino functional silicones, meaning a silicone containing at least one primary, secondary or tertiary amine group, or a quaternary ammonium group. Examples of suitable amino functional silicones 15 include polysiloxanes having the CTFA designation "amodimethicone." Also suitable for use in compositions of this invention are silicone gums having a slight degree of crosslinking, as are described for example in WO 96/31188. Preferably, the silicone compound comprises dimethicone, dimethiconol, amodimethicone, derivatives or mixtures thereof. More preferably, the silicone compound comprises dimethiconol, dimethicone, derivatives or a mixture thereof, more preferably comprises dimethiconol.

It is preferred that the composition of the present invention comprises 0.5 to 5%, preferably from 1 to 3%, more preferably from 1.3 to 2.5% of said silicone by weight of the composition.

### Cleansing surfactant

### Cleansing surfactant

The composition in accordance with this invention comprises a cleansing surfactant comprising an anionic surfactant.

Non-limiting examples of suitable anionic surfactants are alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule. Typical anionic surfactants for use in compositions of the invention include, but not limited to, sodium oleyl succinate, ammonium lauryl sulphosuccinate, sodium lauryl sulphate, sodium lauryl ether sulphate, sodium lauryl ether sulphosuccinate, ammonium lauryl sulphate, sodium dodecylbenzene sulphonate, triethanolamine dodecylbenzene sulphonate, sodium cocoyl isethionate, sodium lauryl isethionate, lauryl ether carboxylic acid, sodium N-lauryl sarcosinate or mixtures thereof.

Preferred anionic surfactants are the alkyl sulphates and alkyl ether sulphates. Preferred alkyl sulphates are C8-18 alky sulphates, more preferably C12-18 alkyl sulphates, preferably in the form of a salt with a solubilising cation such as sodium, potassium, ammonium or substituted ammonium. Examples are sodium lauryl sulphate (SLS) or sodium dodecyl sulphate (SDS). It is particularly preferred that the anionic surfactant is alkyl ether sulphate. Preferred alkyl ether sulphates are those having the formula: RO(CH2CH2O)nSO¬3M; wherein R is an alkyl or alkenyl having from 8 to 18 (preferably 12 to 18) carbon atoms; n is a number having an average value of greater than at least 0.5, preferably between 1 and 3, more preferably between 1 and 2; and M is a solubilising cation such as sodium, potassium, ammonium or substituted ammonium. An example is sodium lauryl ether sulphate (SLES). Preferred alkyl ether sulphate is sodium lauryl ether sulphate having an average degree of ethoxylation of from 0.5 to 3, preferably from 1 to 3, more preferably from 1 to 2.

It is preferred that the anionic surfactant is an ethoxylated alkyl sulphate a degree of ethoxylation of less than 3, preferably an ethoxylated alkyl sulphate a degree of ethoxylation of less than 2, more preferably sodium laureth sulphate (1EO).

The anionic surfactants are typically present in hair care composition of the present invention at a level of from 0.5 to 20%, more preferably from 2 to 16% and most preferably from 3 to 16%, by weight of the hair care composition.

Suitable amphoteric surfactant examples include alkyl amine oxides, alkyl betaines, alkyl amidopropyl betaines, alkyl sulphobetaines (sultaines), alkyl amphoacetates, alkyl amphopropionates, alkyl amidopropyl hydroxysultaines, wherein the alkyl group has from 8 to 19 carbon atoms.

Preferably, the amphoteric surfactant is a betaine surfactant. It is preferred that the betaine surfactant is one of alkyl amidopropyl betaines. Cocamidopropyl betaine (CAPB) is particularly preferred.

In a preferred embodiment, the composition comprises from 0.1 to 10 wt.%, preferably from 0.5 to 8 wt.%, more preferably from 1 to 5 wt.% of an amphoteric surfactant by weight of the total composition.

The ratio of ethoxylated anionic surfactant to amphoteric surfactant in which the ratio of anionic surfactant to amphoteric surfactant is less than 10:1, preferably from 2:1 to 10:1, more preferably from 3:1 to 9:1.

The total amount of cleansing surfactant in a shampoo composition for use in the invention is generally from 3 to 35 wt%, preferably from 5 to 25 wt%, most preferably from 7 to 16 wt% of the total composition.

### Anti-dandruff agent

It is preferred that the hair care composition of the present invention comprises an anti-dandruff agent. Suitable anti-dandruff agents that may be used in this invention are selected from zinc pyrithione, piroctone olamine (Octopirox^{®}), azole based anti-fungal agents, selenium sulfide and mixtures thereof. The preferred azole based anti-fungal agent is ketoconazole, climbazole or mixtures thereof. Preferably, the anti-dandruff agent is selected from zinc pyrithione, climbazole and mixtures thereof. In another preferred embodiment, the anti-dandruff agent is piroctone olamine, climbazole, and mixtures thereof.

The hair care composition of the invention may comprise the anti-dandruff agent in an amount of from 0.01 to 10%, more preferably from 0.05 to 5%, more preferably still from 0.1 to 2%, and most preferably from 0.2 to 1.5%, by weight of the hair care composition.

### Hair care composition

The hair care composition in accordance with the present invention comprises an aqueous cosmetically acceptable carrier.

In a preferred embodiment, the hair care composition is a shampoo, particularly a rinse off shampoo. Shampoo compositions for use in the invention are generally aqueous, i.e. they have water or an aqueous solution or a lyotropic liquid crystalline phase as their major component. Suitably, the shampoo composition will comprise from 50 to 98%, preferably from 60 to 92% water by weight based on the total weight of the composition. Such compositions are referred to as having an aqueous base.

The composition of the present invention may additionally comprise a cationic polymer

The composition may also comprise a cationic modified guar polymer having a molecular weight of from 0.3 to 2.5 million Dalton and a cationic degree of substitution of from 0.05 to 0.4.

It is preferred that the cationic modified guar polymer has a molecular weight of from 1.0 to 2.3 million Dalton, more preferably from 1.0 to 1.5 million Dalton.

It is preferred that the cationic guar polymer has a cationic degree of substitution of from 0.1 to 0.3, more preferably from 0.16 to 0.2.

It is most preferred that cationic modified guar polymer has a molecular weight of from 1.0 million to 1.5 million Dalton and a cationic degree of substitution (DS) of from 0.16 to 0.20. This DS value corresponds to a charge density of from 0.85 to 1.05.

Cationic guar polymer is preferably guar hydroxypropyltrimonium chloride. Guar polymer predominantly contains galactomannan polymer chains. This polymer is available at various molecular weights and degree of cationic substitutions depending on how much the guar has been hydrolysed and cationised.

Generally for cationic polysaccharide polymers, the hydroxyl groups of the non-modified monomeric sugar ring units are the sites for cationic substitution. Degree of substitution (DS) is typically in the range from 0 to 3 due to the fact that the monomeric sugar unit of most polysaccharide has in average three hydroxyl groups available for substitution. In addition to the DS, the cationic charge on polymers can also be quantified as cationic charge density. DS has previously been determined by different methods. For example, the cationic charge density of the polymer has in some cases been calculated based on a percent nitrogen content determined via the Kjeldahl method as described in US Pharmacopoeia under chemical tests for nitrogen determination and is expressed in milliequivalents (meq) per gram. The cationic charge density of the polymer in the present invention is, however, calculated from the degree of substitution, which is measured by 1H NMR in a solvent of deuterium oxide (D2O) and deuterium chloride (DCI) mixture.

In many cases the DS obtained from 1H NMR measurement may not be suitable to be compared with that obtained from Kjeldahl method, due to the fact that the two methods are influenced by different factors.

In the wide spectrum of molecular weights of guars available, the cationic guar for use in the present invention has a molecule weight which is considered a 'medium' molecular weight. In the wide range of charge densities, the above range for use in the present invention is considered a 'medium' range. The cationic modified guar deposition polymer is preferably present in an amount of from 0.001 to 2%, more preferably from 0.01 to 1%, in 0.04 to 0.5%, more preferably 0.08 to 0.25% by weight of the composition.

The compositions of the present invention may comprise a copolymer acrylamidopropyltrimonium chloride. Preferably, the copolymer also comprises acrylamide in addition to the acrylamidopropyltrimonium chloride. Particularly preferred copolymer is a copolymer of acrylamidopropyltrimonium chloride and acrylamide.

The copolymer according to the present invention preferably has a charge density of from 1.0 to 3.0 meq per gram (meq/g), more preferably from 1.1 to 2.5 meq/g, more preferably still from 1.2 to 2.5 meq/g and most preferably from 1.3 to 2.5 meq/g. The copolymer preferably has a molecular weight of from 100,000 to 3,000,000 gram per mole (g/mol), more preferably from 500,000 to 2,800,000 g/mol, more preferably still from 800,000 to 2,500,000 g/mol, most preferably from 1,000,000 to 2,500,000 g/mol. An example of a suitable copolymer is commercially available from Ashland under the trade name N-Hance SP-100^{®}. N-Hance SP-100^{®} has a charge density of from 1.8 to 2.2 meq/g and a molecular weight of from 1,800,000 to 2,200,000 g/mol.

The hair care composition of the present invention may typically comprise the copolymer in an amount of from 0.001 to 2%, more preferably from 0.01 to 1%, even more preferably from 0.01 to 0.8% and most preferably from 0.05 to 0.5% by weight of the composition.

The water-insoluble conditioning agent may include non-silicone conditioning agent comprising non-silicone oily or fatty materials such as hydrocarbon oils, fatty esters and mixtures thereof. Preferably the composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

The suspending agent is generally present in hair care composition of this invention in an amount of from 0.1 to 10%, more preferably from 0.2 to 6%, and most preferably from 0.3 to 4%, based on total weight of the hair care composition and including all ranges subsumed therein.

Preservatives may also be incorporated into the hair care composition of this invention to protect against the growth of potentially harmful microorganisms. Suitable traditional preservatives include alkyl esters of parahydroxybenzoic acid, hydantoin derivatives, propionate salts, and a variety of quaternary ammonium compounds. Illustrative yet non-limiting examples of the types of preservatives that may be used in this invention include, for examples, phenoxyethanol, sodium salicylate, methyl paraben, butyl paraben, propyl paraben, diazolidinyl urea, sodium dehydroacetate, benzyl alcohol, sodium benzoate, iodopropynyl butylcarbamate, caprylyl glycol, disodium EDTA or mixtures thereof. In an especially preferred embodiment, the preservative is sodium benzoate, phenoxyethanol, sodium salicylate or a mixture thereof. Preservatives are preferably employed in amounts ranging from 0.01 to 2% by weight of the hair care composition.

The hair care composition of the present invention may contain other ingredients which are common in the art to enhance physical properties and performances. Suitable ingredients include but are not limited to fragrance, dyes and pigments, pH adjusting agents, pearlescers or opacifiers, viscosity modifiers, thickeners, and natural hair nutrients such as botanicals, fruit extracts, sugar derivatives and amino acids.

### Method and Use

The present invention provides for a non-therapeutic method of treating the hair or scalp comprising the step of application of a composition of the first aspect onto hair or scalp. The method is non-therapeutic in nature. Preferably it is for cosmetic purpose.

The present invention also provides for a non-therapeutic method of delivering oil control sensory, preferably reducing greasiness, onto hair or scalp comprising the step of applying the hair care composition of the first aspect onto hair or scalp surfaces of an individual followed by rinsing the surfaces with water. The method is non-therapeutic in nature. Preferably it is for cosmetic purpose.

The present invention also provides non-therapeutic use of a composition of the first aspect for delivering oil control sensory, preferably reducing greasiness, on hair or scalp.

Preferably, the present invention also provides non-therapeutic use of a composition of the first aspect for delivering oil control sensory, preferably reducing greasiness, meanwhile preventing or alleviating the symptoms of dandruff on the scalp or hair.

### Examples

### Example 1

This example demonstrated the combination of cationic cellulose and small sized silicone compound within specific ratio range can deliver oil control sensory to hair or scalp. The compositions according to the formulations detailed in Table 1 are prepared. All ingredients are expressed by weight percent of the total formulation, and as level of active ingredient.

**TABLE 1**

| **Ingredient** | **Samples** | | |
|---|---|---|---|
| | **A** | **B** | **1** |
| Water | To 100 | To 100 | To 100 |
| Sodium lauryl sulphate | 14 | 14 | 14 |
| Cocoamidopropyl betaine | 1.60 | 1.60 | 1.60 |
| PQ 10 (JR30M) | - | - | 0.30 |
| Dimethiconol (Mean diameter D50: 150-230nm) | 0.78 | 1.30 | 1.30 |
| Dimethicone (Mean diameter D50: 7-11microns) | 0.52 | - | - |
| Carbomer | 0.60 | 0.60 | 0.60 |
| Guar hydroxypropyltrimonium chloride | 0.20 | 0.20 | 0.20 |
| Preservative | 0.80 | 0.80 | 0.80 |
| Propylene Glycol | 0.48 | 0.48 | 0.48 |
| Sodium chloride | 2.00 | 2.00 | 2.00 |
| Sodium Hydroxide | 0.50 | 0.50 | 0.50 |
| Zinc pyrithione | 1.00 | 1.00 | 1.00 |
| Climbazole | 0.48 | 0.48 | 0.48 |
| Weight ratio of cationic cellulose to small sized silicone | - | - | 4.33:1 |

The compositions (Sample A vs. Sample B, Sample B vs. Sample 1) in Table 1 were used by 30 female consumers (50%normal hair and 50%greasy hair) and scored.

The results showed that Sample 1 was significantly (at least 95% conf.) higher in the following attributes comparing with Sample B:
Clean & Fresh properties:
- More clean feel on hair
- More effectively to remove greasiness
- Less greasiness

The results showed that Sample B and Sample A are comparable in the following attributes: Clean & Fresh properties:
- More clean feel on hair
- More effectively to remove greasiness
- Less greasiness

It can be concluded from the results that Sample 1 comprising cationic cellulose and small sized silicone compound with the specific weight ratio within the scope of the present invention can enhance the oil control sensorial experience especially removing greasiness, compared to Sample A and B (outside the scope of the present invention). It also indicates that the increasing of amount of small sized silicone without the cationic cellulose within the scope cannot deliver desired sensory experience (Comparing Sample B to Sample A).

### Example 2

This example demonstrated the combination of cationic cellulose and small sized silicone compound within weight ratio outside the scope of the present invention cannot deliver oil control sensory to hair or scalp. The compositions according to the formulations detailed in Table 2 are prepared. All ingredients are expressed by weight percent of the total formulation, and as level of active ingredient.

**TABLE 2**

| **Ingredient** | **Samples** | |
|---|---|---|
| | **C** | **D** |
| Water | To 100 | To 100 |
| Sodium lauryl sulphate | 14 | 14 |
| Cocoamidopropyl betaine | 1.60 | 1.60 |
| PQ 10 (JR30M) | - | 0.30 |
| Dimethiconol (Mean diameter D50: 150-230nm) | 1.20 | 1.20 |
| Dimethicone (Mean diameter D50: 7-11microns) | 0.80 | 0.80 |
| Carbomer | 0.60 | 0.60 |
| Guar hydroxypropyltrimonium chloride | 0.20 | 0.20 |
| Preservative | 0.80 | 0.80 |
| Propylene Glycol | 0.48 | 0.48 |
| Sodium chloride | 2.00 | 2.00 |
| Sodium Hydroxide | 0.50 | 0.50 |
| Zinc pyrithione | 1.00 | 1.00 |
| Climbazole | 0.48 | 0.48 |
| Weight ratio of cationic cellulose to small sized silicone | - | 4:1 |

The compositions (Sample C vs. Sample D) in Table 2 were used by 30 female consumers (50%normal hair and 50% greasy hair) and scored.

The results showed that Sample D with Sample C were comparable in the following attributes: Clean & Fresh properties:
- More clean feel on hair
- More effectively to remove greasiness
- Less greasiness

The result above demonstrates that inclusion of cationic cellulose and small sized silicone compound with the specific weight ratio outside the scope of the present invention (Sample D) cannot deliver desired sensory experience.

## Claims

1. A hair care composition comprising:
i) a cleansing surfactant comprising an anionic surfactant;
ii) a cationic cellulose; and
iii) a silicone compound having mean particle size from 150 to 450nm; wherein the weight ratio of the amount of said silicone compound to the amount of said cationic cellulose is from 4.3:1 to 10:1; wherein the mean particle size of the silicone compound is measured by means of a laser light scattering technique.

2. The hair care composition as claimed in claim 1 wherein said cationic cellulose is Polyquaternium 10.

3. The hair care composition as claimed in claim 1 or 2 wherein said silicone compound comprises dimethiconol, dimethicone, amodimethicone, derivatives or mixtures thereof thereof, preferably comprises dimethiconol, dimethicone, derivatives or a mixture thereof, more preferably comprises dimethiconol.

4. The hair care composition as claimed in any of claims 1 to 3 wherein said composition comprises 0.2 to 1%, preferably 0.3 to 1%, more preferably from 0.3 to 0.6% of said cationic cellulose by weight of the composition.

5. The hair care composition as claimed in any of claims 1 to 4 wherein said composition comprises 0.5 to 5%, preferably from 1 to 3%, more preferably from 1.3 to 2.5% of said silicone by weight of the composition.

6. The hair care composition as claimed in any of claims 1 to 5 wherein said composition comprise an antidandruff agent, selected from zinc pyrithione, piroctone olamine, azole based anti-fungal agents, selenium sulfide and mixtures thereof.

7. The hair care composition as claimed in claim 6 wherein said composition comprises 0.01 to 10%, preferably 0.05 to 5%, more preferably from 0.1 to 2%, and most preferably from 0.2 to 1.5% of said antidandruff agent by weight of the composition.

8. The hair care composition as claimed in any of claims 1 to 7 wherein said anionic surfactant is an ethoxylated alkyl sulphate a degree of ethoxylation of less than 3, preferably sodium lauryl ether sulphate.

9. The hair care composition as claimed in any of claims 1 to 8 wherein said composition additionally comprises a cationic polymer.

10. The hair care composition as claimed in claim 9 wherein said composition comprises a cationic guar polymer, preferably guar hydroxypropyltrimonium chloride.

11. The hair care composition as claimed in any of claims 1 to 10 wherein said composition comprises an amphoteric surfactant, preferably a betaine surfactant, more preferably one of alkyl amidopropyl betaines.

12. The hair care composition as claimed in any of claims 1 to 11 wherein said composition is a shampoo.

13. A non-therapeutic method of treating the hair or scalp comprising the step of applying a composition as claimed in any of claims 1 to 12 to the hair or scalp.

14. A non-therapeutic method of delivering oil control sensory comprising the step of applying the hair care composition as claimed in any of claims 1 to 12 onto hair or scalp surfaces of an individual followed by rinsing the surfaces with water.

15. Non-therapeutic use of a composition as claimed in any of claims 1 to 12 for delivering oil control sensory on hair or scalp.

## Patentansprüche

1. Haarpflegezusammensetzung, umfassend:
i) ein Reinigungstensid, das ein anionisches Tensid umfasst;
ii) eine kationische Cellulose; und
iii) eine Silikonverbindung mit einer mittleren Partikelgröße von 150 bis 450 nm;
wobei das Gewichtsverhältnis der Menge der Silikonverbindung zur Menge der kationischen Cellulose 4,3:1 bis 10:1 beträgt; wobei die mittlere Partikelgröße der Silikonverbindung mittels einer Laserlichtstreuungstechnik gemessen wird.

2. Haarpflegezusammensetzung, wie im Anspruch 1 beansprucht, wobei die kationische Cellulose Polyquaternium 10 ist.

3. Haarpflegezusammensetzung, wie im Anspruch 1 oder 2 beansprucht, wobei die Silikonverbindung Dimethiconol, Dimethicone, Amodimethicone, Derivate oder Mischungen davon umfasst, vorzugsweise Dimethiconol, Dimethicone, Derivate oder eine Mischung davon umfasst, bevorzugter Dimethiconol umfasst.

4. Haarpflegezusammensetzung, wie in einem der Ansprüche 1 bis 3 beansprucht, wobei die Komposition 0,2 bis 1%, vorzugsweise 0,3 bis 1%, bevorzugter 0,3 bis 0,6 % der kationischen Cellulose, bezogen auf das Gewicht der Zusammensetzung, umfasst.

5. Haarpflegezusammensetzung, wie in einem der Ansprüche 1 bis 4 beansprucht, wobei die Zusammensetzung 0,5 bis 5%, vorzugsweise 1 bis 3%, bevorzugter 1,3 bis 2,5% des Silikons, bezogen auf das Gewicht der Zusammensetzung, umfasst.

6. Haarpflegezusammensetzung, wie in einem der Ansprüche 1 bis 5 beansprucht, wobei die Zusammensetzung ein Antischuppenmittel umfasst, das unter Zinkpyrithion, Piroctonolamin, Azol-basierten Antimykotika, Selensulfid und Mischungen davon ausgewählt ist.

7. Haarpflegezusammensetzung, wie im Anspruch 6 beansprucht, wobei die Zusammensetzung 0,01 bis 10%, vorzugsweise 0,05 bis 5%, bevorzugter 0,1 bis 2% und höchst bevorzugt 0,2 bis 1,5% des Antischuppenmittels, bezogen auf das Gewicht der Zusammensetzung, umfasst.

8. Haarpflegemittel, wie in einem der Ansprüche 1 bis 7 beansprucht, wobei das anionische Tensid ein ethoxyliertes Alkylsulfat mit einem Ethoxylierungsgrad von weniger als 3, vorzugsweise Natriumlaurylethersulfat, ist.

9. Haarpflegezusammensetzung, wie in einem der Ansprüche 1 bis 8 beansprucht, wobei die Zusammensetzung zusätzlich ein kationisches Polymer umfasst.

10. Haarpflegezusammensetzung, wie im Anspruch 9 beansprucht, wobei die Zusammensetzung ein kationisches Guarpolymer, vorzugsweise Guarhydroxypropyltrimoniumchlorid, umfasst.

11. Haarpflegezusammensetzung, wie in einem der Ansprüche 1 bis 10 beansprucht, wobei die Zusammensetzung ein amphoteres Tensid, vorzugsweise ein Betain-Tensid, bevorzugter eines der Alkylamidopropylbetaine, umfasst.

12. Haarpflegezusammensetzung, wie in einem der Ansprüche 1 bis 11 beansprucht, wobei die Zusammensetzung ein Shampoo ist.

13. Nicht-therapeutisches Verfahren zur Behandlung des Haares oder der Kopfhaut, umfassend den Schritt des Aufbringens einer Zusammensetzung, wie in einem der Ansprüche 1 bis 12 beansprucht, auf das Haar oder die Kopfhaut.

14. Nicht-therapeutisches Verfahren zur Vermittlung sensorischer Ölkontrolle, umfassend den Schritt des Aufbringens der Haarpflegezusammensetzung, wie in einem der Ansprüche 1 bis 12 beansprucht, auf Haar- oder Kopfhautoberflächen einer Person, gefolgt vom Abspülen der Oberflächen mit Wasser.

15. Nicht-therapeutische Verwendung einer Zusammensetzung, wie in einem der Ansprüche 1 bis 12 beansprucht, zur Vermittlung sensorischer Ölkontrolle auf dem Haar oder der Kopfhaut.

## Revendications

1. Composition de soin capillaire comprenant :
i) un tensioactif nettoyant comprenant un tensioactif anionique ;
ii) une cellulose cationique ; et
iii) un composé siliconé ayant une granulométrie moyenne de 150 à 450 nm ;
dans laquelle le rapport en poids de la quantité dudit composé siliconé à la quantité de ladite cellulose cationique est de 4,3/1 à 10/1 ; dans laquelle la granulométrie moyenne du composé siliconé est mesurée au moyen d'une technique de diffusion de lumière laser.

2. Composition de soin capillaire selon la revendication 1, dans laquelle ladite cellulose cationique est le polyquaternium 10.

3. Composition de soin capillaire selon la revendication 1 ou 2, dans laquelle ledit composé siliconé comprend le diméthiconol, la diméthicone, l'amodiméthicone, leurs dérivés ou mélanges, de préférence comprend le diméthiconol, la diméthicone, leurs dérivés ou mélanges, mieux encore comprend le diméthiconol.

4. Composition de soin capillaire selon l'une quelconque des revendications 1 à 3, laquelle composition comprend 0,2 à 1 %, de préférence 0,3 à 1 %, mieux encore 0,3 à 0,6 % de ladite cellulose cationique en poids de la composition.

5. Composition de soin capillaire selon l'une quelconque des revendications 1 à 4, laquelle composition comprend 0,5 à 5 %, de préférence 1 à 3 %, mieux encore 1,3 à 2,5 % de ladite silicone en poids de la composition.

6. Composition de soin capillaire selon l'une quelconque des revendications 1 à 5, laquelle composition comprend un agent antipelliculaire choisi parmi la pyrithione de zinc, la piroctone olamine, les agents antifongiques à base d'azole, le sulfure de sélénium et leurs mélanges.

7. Composition de soin capillaire selon la revendication 6, laquelle composition comprend 0,01 à 10 %, de préférence 0,05 à 5 %, mieux encore 0,1 à 2 %, et tout spécialement 0,2 à 1,5 % dudit agent antipelliculaire en poids de la composition.

8. Composition de soin capillaire selon l'une quelconque des revendications 1 à 7, dans laquelle ledit tensioactif anionique est un alkylsulfate éthoxylé ayant un degré d'éthoxylation inférieur à 3, de préférence est le lauryléther-sulfate de sodium.

9. Composition de soin capillaire selon l'une quelconque des revendications 1 à 8, laquelle composition comprend de plus un polymère cationique.

10. Composition de soin capillaire selon la revendication 9, laquelle composition comprend un polymère de guar cationique, de préférence le chlorure d'hydroxypropyltrimonium-guar.

11. Composition de soin capillaire selon l'une quelconque des revendications 1 à 10, laquelle composition comprend un tensioactif amphotère, de préférence un tensioactif bétaïne, mieux encore l'une des alkylamidopropyl-bétaïnes.

12. Composition de soin capillaire selon l'une quelconque des revendications 1 à 11, laquelle composition est un shampooing.

13. Procédé non thérapeutique de traitement des cheveux ou du cuir chevelu, comprenant l'étape d'application d'une composition de l'une quelconque des revendications 1 à 12 sur les cheveux ou le cuir chevelu.

14. Procédé non thérapeutique de délivrance d'une expérience sensorielle de contrôle du sébum, comprenant l'étape d'application de la composition de soin capillaire de l'une quelconque des revendications 1 à 12 sur des surfaces des cheveux ou du cuir chevelu d'un individu, suivie d'un rinçage à l'eau des surfaces.

15. Utilisation non thérapeutique d'une composition de l'une quelconque des revendications 1 à 12 pour délivrer une expérience sensorielle de contrôle du sébum sur les cheveux ou le cuir chevelu.
